Europäisches Patentamt

European Patent Office

Office européen des brevets

(19)

(11) Veröffentlichungsnummer: **0 098 475**
A2

# (12) EUROPÄISCHE PATENTANMELDUNG

(21) Anmeldenummer: 83106168.4

(22) Anmeldetag: 24.06.83

(51) Int. Cl.³: **A 61 K 31/615**

(30) Priorität: 08.07.82 DE 3225491

(43) Veröffentlichungstag der Anmeldung: **18.01.84**
**Patentblatt 84/3**

(84) Benannte Vertragsstaaten: **AT BE CH DE FR GB IT LI LU NL SE**

(71) Anmelder: **Beiersdorf Aktiengesellschaft, Unnastrasse 48, D-2000 Hamburg 20 (DE)**

(72) Erfinder: **Dies, Rainer, Dr., Liebermannstrasse 48, D-2000 Hamburg 52 (DE)**
Erfinder: **Asmussen, Bodo, Dr., Dorotheenweg 1a, D-2071 Ammersbeck (DE)**

(54) Pharmazeutische Zusammensetzung, ihre Herstellung und Verwendung.

(57) Pharmazeutische Zusammensetzung, enthaltend Xipamid und alpha-Methyldopa zur antihypertensiven Behandlung.

EP 0 098 475 A2

0098475

Beiersdorf Aktiengelllschaft
Hamburg

Pharmazeutische Zusammensetzung,
ihre Herstellung und Verwendung

Gegenstand der Erfindung ist eine neue pharmazeutische Zusammensetzung, ihre Herstellung und Verwendung.

Das 4-Chlor-5-sulfamoyl-salicylsäure-(2,6-dimethylanilid) ist als Xipamid bekannt und als Diuretikum eingeführt, vgl. US-PS 3 567 777.

L-3-(3,4-Dihydroxyphenyl)-2-methylalanin ist als alpha-Methyldopa bekannt und ein anerkanntes Antihypertonikum, vgl. US-PS 2 868 818.

Xipamid und alpha-Methyldopa werden in der Humanmedizin seit vielen Jahren aufgrund ihrer diuretischen und antihypertensiven Eigenschaften verwendet.

Kombinationspräparate mit alpha-Methyldopa sind bereits bekannt, aber nicht die Kombination mit Xipamid.

Es wurde nun überraschend gefunden, daß eine pharmazeutische Zusammensetzung, die als Wirkstoff Xipamid und alpha-Methyldopa enthält, außerordentlich günstige pharmakologische und toxikologische Eigen-

0098475

-2-

schaften aufweist. Diese Zusammensetzung kann insbesondere als antihypertensives Arzneimittel verwendet werden.

Xipamid ist ein Diuretikum beziehungsweise Saluretikum von starker und mittellang andauernder Wirkung.

Alpha-Methyldopa ist ein Antihypertensivum, das die Natrium- und Wasserausscheidung verringert. Aufgrund dieser pharmakologischen Eigenschaften kann es unter alpha-Methyldopa zu unerwünschter Wasseransammlung im Gewebe (Ödem) kommen. Durch die gleichzeitige Gabe des Xipamids wird dies verhindert. Die Verwendung der erfindungsgemäßen Zusammensetzung, die Xipamid und alpha-Methyldopa enthält, erlaubt daher in unerwarteter Weise eine wirksame antihypertensive Behandlung über einem längeren Zeitraum durchzuführen, ohne daß es zu unerwünschten Wasseransammlungen im Gewebe kommt. Dieser überragende pharmakologische Effekt war aus dem Stand der Technik nicht herleitbar.

Zudem wurde in nicht vorhersehbarer Weise gefunden, daß die antihypertensive Wirksamkeit der Kombination, insbesondere bei den Gewichtsverhältnissen von 1 Teil Xipamid pro 10 bis 45 Teilen alpha-Methyldopa und vorzugsweise 1 Teil Xipamid pro 30 Teilen alpha-Methyldopa, besonders günstig war, in dem es zu einer Verstärkung der antihypertensiven Wirkung über das bei den Einzelsubstanzen beobachtete Maß hinaus kam.

-3-

Ferner wurden Hinweise darauf gefunden, daß die Toxizität der erfindungsgemäßen Mischung geringer ist als diejenige, die man aufgrund der Toxizitäten der Bestandteile erwarten konnte.

Mit diesen hervorragenden Eigenschaften sind die erfindungsgemäßen pharmazeutischen Zusammensetzungen, die Xipamid und alpha-Methyldopa enthalten, zur Verwendung in der Humanmedizin, vorzugsweise bei Hypertonie, insbesondere in der Langzeitbehandlung der arteriellen Hypertonie, besonders geeignet.

Bevorzugt enthalten die erfindungsgemäßen pharmazeutischen Zusammensetzungen 1 Gewichtsteil Xipamid und 1 bis 45, vorzugsweise 10 bis 45, Gewichtsteile alpha-Methyldopa. Besonders bevorzugt wird eine Zusammensetzung mit 1 Gewichtsteil Xipamid und etwa 30 Gewichtsteilen alpha-Methyldopa.

Beim Erwachsenen verabreicht, kann die Tagesdosis der oral verabreichten Wirkstoffe beispielsweise etwa 10 bis 30 mg Xipamid und entsprechende Mengen alpha-Methyldopa, z.B. 300-900 mg alpha-Methyldopa, betragen, vorzugsweise etwa 10 mg Xipamid und 300 mg alpha-Methyldopa bis etwa 20 mg Xipamid und 600 mg alpha-Methyldopa, insbesondere aber 20 mg Xipamid und 600 mg alpha-Methyldopa.

Die erfindungsgemäßen pharmazeutischen Zusammensetzungen können parenteral oder über den Verdauungstrakt verabreicht werden, wobei die orale Verabreichung bevorzugt ist.

-4-

Diese pharmazeutischen Zusammensetzungen können beispielsweise fest oder flüssig sein und in den bei der Humanmedizin üblicherweise verwendeten pharmazeutischen Formen vorliegen, beispielsweise als Tabletten, Dragees, Gelkügelchen, Granulat, trinkbare Suspension, Suppositorien, injizierbare Lösung, deren Herstellung nach den üblichen Verfahren erfolgt. Zu den Wirkstoffen können die bei diesen pharmazeutischen Zusammensetzungen üblicherweise verwendeten Exzipienten hinzugefügt werden, beispielsweise Talkum, kolloidales Siliciumdioxid, Gummi arabicum, Lactose, Stärke, Magnesiumstearate, Kakaobutter, wässrige oder nicht wässrige Vehikel, Fette tierischen oder pflanzlichen Ursprungs, paraffinische Derivate, Glykole, verschiedene Netz-, Dispergier- oder Emulgiermittel und/ oder Konservierungsmittel.

Das nachstehende Beispiel erläutert die Erfindung, ohne sie jedoch einzuschränken.

Beispiel
Pharmazeutische Zusammensetzung

Man stellt Tabletten her, die der folgenden Formulierung entsprechen:

| Xipamid | 10 mg |
|---|---|
| alpha-Methyldopa | 300 mg |
| Exzipient (q.s.) | 450 mg |

(Exzipient: Aerosil, Maisstärke, behandelte Stärke, Lactose, Magnesiumstearat, Talkum).

Patentansprüche

1. Pharmazeutische Zusammensetzung, enthaltend Xipamid und alpha-Methyldopa.

2. Pharmazeutische Zusammensetzung gemäß Anspruch 1, enthaltend 1 Gewichtsteil Xipamid und 1 bis 45 Gewichtsteile alpha-Methyldopa.

3. Pharmazeutische Zusammensetzung gemäß Anspruch 1 oder 2, enthaltend 1 Gewichtsteil Xipamid und etwa 30 Gewichtsteile alpha-Methyldopa.

4. Pharmazeutische Zusammensetzung gemäß einem der Ansprüche 1 bis 3, enthaltend für die orale Verabreichung geeignete pharmazeutische Trägersubstanzen.

5. Pharmazeutische Zusammensetzungen gemäß einem der Ansprüche 1 bis 4 in Form von Tabletten oder Dragees, enthaltend 10 mg Xipamid und 300 mg alpha-Methyldopa.

6. Pharmazeutische Zusammensetzung nach einem der der Ansprüche 1 bis 5 zur Anwendung bei Hypertonie.

7. Verfahren zur Herstellung der pharmazeutischen Zusammensetzung nach einem der Ansprüche 1 bis 5, dadurch gekennzeichnet, daß man Xipamid mit alpha-Methyldopa gegebenenfalls zusammen mit einem pharmazeutischen Träger zu einem pharmazeutischen Präparat verarbeitet.